# EUROPÄISCHE PATENTSCHRIFT

(11) **EP 3 352 763 B1**
(45) Veröffentlichungstag und Bekanntmachung des Hinweises auf die Patenterteilung: **30.03.2022**
(21) Anmeldenummer: 16769982.6
(22) Anmeldetag: 20.09.2016
(51) Int. Cl.: A61K 31/7048, A61P 31/16

(54) **SUBSTANZ ZUR PROPHYLAXE UND BEHANDLUNG VON INFEKTIONEN DURCH INFLUENZAVIREN**
SUBSTANCE FOR PROPHYLAXIS AND TREATMENT OF INFECTIONS BY INFLUENZA VIRUSES
SUBSTANCE POUR LA PROPHYLAXIE ET LE TRAITEMENT D'INFECTIONS PROVOQUÉES PAR DES VIRUS INFLUENZA

(30) Priorität: 21.09.2015 DE 102015115876
(43) Veröffentlichungstag der Anmeldung: 01.08.2018
(73) Patentinhaber: Universität Hamburg, 20148 Hamburg (DE); Atriva Therapeutics GmbH, 72072 Tübingen (DE)
(72) Erfinder: PLANZ, Oliver, 72581 Dettingen/Erms (DE); HARTMAYER, Carmen, 72379 Hechingen (DE); ROHN, Sascha, 10629 Berlin (DE); RIEHLE, Peer, 20257 Hamburg (DE)
(74) Vertreter: Schiweck Weinzierl Koch Patentanwälte Partnerschaft mbB
(86) Internationale Anmeldenummer: PCT/EP2016/072219
(87) Internationale Veröffentlichungsnummer: WO 2017/050717

(56) Entgegenhaltungen:
- WO-A1-2009/151173
- Peer Riehle: "Phenolische Inhaltsstoffe in Cistus incanus Tee - Charakterisierung und Stabilität innerhalb der Teezubereitung Dissertation", , 1. August 2014 (2014-08-01), XP055324735, Gefunden im Internet: URL:http://ediss.sub.uni-hamburg.de/vollte xte/2014/7074/pdf/Dissertation.pdf [gefunden am 2016-11-30]
- MITROCOTSA D ET AL: "Evaluation of the antiviral activity of kaempferol and its glycosides against human cytomegalovirus", PLANTA MEDICA, THIEME VERLAG, DE, Bd. 66, Nr. 4, 1. Januar 2000 (2000-01-01) , Seiten 377-379, XP003017069, ISSN: 0032-0943, DOI: 10.1055/S-2000-8550
- Berrin Özçelik ET AL: "Antiviral and Antimicrobial Assessment of Some Selected Flavonoids", Zeitschrift für Naturforschung C, 1. Oktober 2006 (2006-10-01), Seiten 632-638, XP055324543, Germany DOI: 10.1515/znc-2006-9-1003 Gefunden im Internet: URL:https://www.degruyter.com/downloadpdf/ j/znc.2006.61.issue-9-10/znc-2006-9-1003/z nc-2006-9-1003.xml
- SAVOV V M ET AL: "Effects of rutin and quercetin on monooxygenase activities in experimental influenza virus infection", EXPERIMENTAL AND TOXICOLOGIC PATHOLOGY, JENA, DE, Bd. 58, Nr. 1, 15. August 2006 (2006-08-15), Seiten 59-64, XP028021840, ISSN: 0940-2993, DOI: 10.1016/J.ETP.2006.05.002 [gefunden am 2006-08-15]
- WEI QIAO ET AL: "Identification of trans-tiliroside as active principle with anti-hyperglycemic, anti-hyperlipidemic and antioxidant effects from", JOURNAL OF ETHNOPHARMACOLOGY, ELSEVIER IRELAND LTD, IE, Bd. 135, Nr. 2, 27. März 2011 (2011-03-27) , Seiten 515-521, XP028203444, ISSN: 0378-8741, DOI: 10.1016/J.JEP.2011.03.062 [gefunden am 2011-04-02]

## Beschreibung

Die Erfindung betrifft eine neue Substanz, nämlich Tilirosid, zur Verwendung in der Prophylaxe und/oder Behandlung von Infektionen durch Influenzaviren, eine diese Substanz aufweisende Zusammensetzung, und damit im Zusammenhang stehende Verwendungen und Verfahren.

Influenzaviren gehören zur Familie der Orthomyxoviridae. Sie sind umhüllte Viren mit einer einzelsträngigen, segmentierten RNA von negativer Polarität. Derzeit sind drei Gattungen bekannt, A, B und C. Die Influenza, auch "echte" Grippe oder Virusgrippe genannt, wird von Viren aus den Gattungen A oder B ausgelöst. Die Influenzaviren und die durch sie ausgelösten Erkrankungen existieren weltweit. Jährlich sind nach Schätzungen der World Health Organization (WHO) 10% bis 20% der Weltbevölkerung betroffen.

Zur Influenzatherapie beim Menschen sind Medikamente aus zwei Substanzklassen zugelassen: Hemmer eines viralen Membranproteins, des sogenannten M2-lonenkanals, wie beispielsweise Amantadin und dessen Derivate, sowie Neuraminidase-Inhibitoren, wie Oseltamivir (Tamiflu) und Zanamivir.

Influenzaviren entwickeln insbesondere gegen Amantadin und Oseltamivir sehr schnell Resistenzen, die an neue infektiöse und resistente Viren weitergegeben werden können. Bereits im Jahr 2009 waren resistente Virusstämme im Umlauf. Saisonale Influenza-A-H1N1 ist zu 96% gegen Oseltamivir und 2% gegen Amantadin resistent. Die Influenza-A-H3N2 weist eine fast 100%-ige Resistenz gegen Amantadin auf.

Die derzeit verfügbaren antiviralen Therapien zeichnen sich außerdem durch eine Vielzahl von Nebenwirkungen aus. So sind für Amantadin Wahrnehmungs- und Stimmungsbeeinträchtigungen, wie Depressionen, Euphorie, Verwirrungszustände, Halluzinationen und Alpträume, Störungen beim Wasserlassen, Schlafstörungen, Störungen der Blutdruckregulation, Übelkeit, Erbrechen und Durchfall beschrieben. Auch für Oseltamivir sind Nebenwirkungen wie Übelkeit, Erbrechen und Magenschmerzen bekannt. Ferner können allergische Reaktionen auftreten sowie eine Verschlechterung bereits bestehender Erkrankungen der Atemwege. Ebenfalls in der Diskussion sind neuropsychiatrische Vorfälle bei Jugendlichen sowie Wechselwirkungen mit anderen Medikamenten.

Die Disserstation von Peer Riehle, Universität Hamburg, 1. August 2014, "Phenolische Inhaltsstoffe in Cistus icanus Tee - Charakterisierung und Stabilität innerhalb der Teezubereitung" wird Tilirosid erwähnt, aber es wird nicht offenbart, dass dieses eine antivirale Wirkung insbesondere gegen Influenzaviren haben könnte.

Vor diesem Hintergrund ist es eine der Erfindung zugrundeliegende Aufgabe, einen neuen Wirkstoff zur Prophylaxe und/oder Behandlung von Infektionen durch Influenzaviren bereitzustellen, mit dem die im Stand der Technik beschriebenen Nachteile der derzeitigen antiviralen Therapien gegen Influenza reduziert oder vorzugsweise vermieden werden können.

Diese Aufgabe wird durch die Bereitstellung von Tilirosid [Kaempferol-3-(6"-trans-p-coumaroyl)glucosid] gelöst.

Tilirosid [Kaempferol-3-(6"-trans-p-coumaroyl)glucosid], auch als Astragalin-6"-trans-p-cumarat oder Potengriffiosid A; 3,4',5,7-Tetrahydroxyflavon-3-(6"-trans-p-cumaroyl)glucosid bezeichnet (CAS-Nr. 20316-62-5), ist ein pflanzliches glycosyliertes und acyliertes Flavonoid. Es hat ein Molekulargewicht von 594,53 g/mol und weist die Summenformel C₃₀H₂₆O₁₃ auf. Es gehört zu der Substanzklasse der Flavonoide. Tilirosid findet sich in höheren Konzentrationen in den Spezies *Castanea sp., Daphne sp., Lamium sp.*, *Platanus sp.*, *Quercus sp.*, *Rosa sp.*, und *Tilia sp.*

Die Erkenntnisse der Erfinder waren überraschend. So ist Tilirosid im Stand der Technik bislang nicht für seine Eignung zur Prophylaxe und Behandlung von Infektionen durch Influenzviren beschrieben.

Die Erfinder konnten in ihren Experimenten feststellen, dass Tilirosid sehr früh in der Infektion mit dem Virus interagieren, nämlich während des Eintritts des Virus und der Freisetzung in die Wirtszelle. Damit ist Tilirosid nach Erkenntnissen der Erfinder die erste Substanz, die den Eintritt bzw. die Freisetzung des Influenzavirus in die Zelle verhindern kann.

Überraschenderweise zeigt Tilirosid auch antivirale Wirksamkeit gegen Influenza-Varianten, die resistent gegen die antivirale Wirkung von Oseltamivir sind.

Tilirosid ist durch niedrige TC₅₀- und EC₅₀-Werte gekennzeichnet, wirkt folglich bereits bei geringen Konzentrationen. Dies hat den Vorteil, dass eine hohe Wirksamkeit bei reduzierten Nebenwirkungen zu erwarten ist.

Wie die Erfinder feststellen konnten, ist Tilirosid hochspezifisch für Influenzaviren. So konnten die Erfinder die Wirksamkeit an verschiedenen Stämmen testen. Gegenüber anderen Viren, wie beispielsweise dem Hantavirus, zeigte sich keine Wirkung.

Unter "Derivaten" von Tilirosid (nicht Gegenstand der Erfindung) werden von letzterem abgeleitete Stoffe mit chemisch ähnlicher Struktur verstanden. Die Derivate weisen ebenfalls die für Tilirosid erfindungsgemäß beobachtete Wirksamkeit, ggf. in verstärkter oder abgeschwächter Form, auf.

Die der Erfindung zugrundeliegende Aufgabe wird hiermit vollkommen gelöst.

| Weiterhin werden offenbart: Gruppe I: | Tilirosid-Isomere; |
|---|---|
| Gruppe II: | Tilirosidderivate mit zusätzlichen Zuckerverbindungen oder alternativen Acylierungen am 3-O-Glucosidrest; |
| Gruppe III: | Tilirosidderivate mit alternativen Substituenten an den C-Atomen; |
| Gruppe IV: | Kaempferol-basierte Derivate, die Zuckerverbindungen an anderen Positionen als Tilirosid oder andere Zuckerverbindungen als Tilirosid an der 3-*O-*Position aufweisen; |
| Gruppe V: | Derivate, die auf Quercetin und Myricetin basieren |

Die Wirkstoffe der Gruppen I-V sind nicht Gegenstand der Erfindung. Diese Maßnahme hat den Vorteil, dass solche Derivate offenbart werden, die nach Erkenntnissen der Erfinder eine mit Tilirosid vergleichbare Wirksamkeit aufweisen können.

Weiterhin sind offenbart: Quercitrin, Quercetin-3-*O*-glucosid, Quercetin-3-*O*-galactosid, Quercitrin, Quercetin-O-xylosid, Rutin und Myricitrin, Myricetin-3-*O*-galactosid, Myricetin-*O*-xylosid. Diese Wirkstoffe sind nicht Gegenstand der Erfindung.

Diese Maßnahme hat den Vorteil, dass dem Fachmann bereits solche Verbindungen offenbart werden, die nach Erkenntnissen der Erfinder vielversprechende Kandidaten für ein wirksames Arzneimittel darstellen können.

Weiterhin werden offenbart: Kaempferol-3-*O*,4'-*O*-diglucosid-7-*O*-rhamnosid, Kaempferol-3-*O*,7-*O*-dirhamnosid, Kaempferol-3-*O*-glucosid, Kaempferol-3-*O*-sambubiosid, Kaempferol-3-*O*-robinosid-7-*O*-glucosid, Kaempferol-7-*O*-neohesperidoside, Kaempferol-3-*O*-rutinoside. Diese Wirkstoffe sind nicht Gegenstand der Erfindung.

Diese Maßnahme hat den Vorteil, dass weitere potenziell geeignete und wirksame Verbindungen offenbart werden. Besonders vorteilhaft können hierbei Kaempferol-3-*O-*glucosid, weil es eine Ausgangsstruktur für weitere mögliche synthetische Derivate der Gruppe III darstellt, Kaempferol-3-*O*-sambubiosid, weil es strukturell sehr nahe am Tilirosid ist, und Kaempferol-3-*O*-rutinoside sein, weil ebenfalls sehr ähnlich zu Tilirosid ist.

Erfindungsgemäß kann dabei das Tilirosid als alleiniger Wirkstoff oder aber alternativ zusammen mit weiteren gegebenenfalls antiviral oder gegen Influenzaviren wirkenden Verbindungen eingesetzt werden.

Ein weiterer Gegenstand der vorliegenden Erfindung betrifft eine Zusammensetzung zur Verwendung in der Prophylaxe und/oder Behandlung von Infektionen durch Influenzaviren, die das erfindungsgemäße Tilirosid aufweist.

Die Merkmale, Eigenschaften und Vorteile des erfindungsgemäßen Tilirosids gelten für die erfindungsgemäße Zusammensetzung gleichermaßen.

Nach einer bevorzugten Weiterbildung ist die erfindungsgemäße Zusammensetzung eine pharmazeutische Zusammensetzung, die zusätzlich eine pharmazeutisch akzeptable Formulierung aufweist.

Pharmazeutisch akzeptable Formulierungen sind dem Fachmann bekannt, beispielsweise aus Remington, The Science and Practice of Pharmacy, 22. Auflage (2012).

Dabei kann das Tilirosid als alleiniger Wirkstoff aber auch in Kombination mit weiteren Wirkstoffen vorgesehen sein.

Nach einer bevorzugten Weiterbildung liegt die erfindungsgemäße Zusammensetzung in flüssiger, fester oder halbfester Form vor. Die erfindungsgemäße Zusammensetzung kann zur oralen, topischen, rektalen oder vaginalen Verabreichung ausgebildet sein. Die erfindungsgemäße Zusammensetzung kann bspw. in Form einer Tablette, Kapsel, eines Aerosols, Mundsprays, einer Mundspülung, Lösung, eines Pulvers, Granulats, Gels, einer Emulsion, Salbe, Paste, Tinktur, Creme ausgestaltet sein.

Diese Maßnahmen haben den Vorteil, dass das erfindungsgemäße Tilirosid bereits in einer solchen Form bereitgestellt wird, die sich für eine optimale Wirksamkeit bewährt hat.

Vorzugsweise wird das Tilirosid und/oder die Zusammensetzung wie folgt verabreicht:
(i) unmittelbar vor der Exposition an dem Influenzavirus, und/oder
(ii) zeitgleich mit der der Exposition an dem Influenzavirus, und/oder
(iii) unmittelbar nach der Exposition an dem Influenzavirus.

Diese Maßnahme nutzt auf vorteilhafte Art und Weise den von den Erfindern erkannten Wirkmechanismus. So wurde die beste antivirale Wirkung festgestellt, wenn die Behandlung zeitgleich mit der Infektion erfolgt. Beginnt die Behandlung unmittelbar vor oder nach der Infektion, ist nach wie vor eine signifikante Reduktion der Viruslast zu beobachten.

"Exposition" bezeichnet die Aussetzung eines zu behandelnden Lebewesens, wie einen Menschen, an eine Influenza aufweisende Umgebung. "Unmittelbar vor der Exposition" bzw. "unmittelbar nach der Exposition" bedeutet erfindungsgemäß, dass die Verabreichung kurz vor bzw. nach einem möglichen Inkontakttreten mit dem Virus erfolgt, vorzugsweise ≤ 10 Stunden, weiter vorzugsweise ≤ 5 Stunden, weiter vorzugsweise ≤ 2 Stunden, weiter vorzugsweise ≤ 1 Stunde erfolgt.

Der von den Erfindern erkannte Wirkmechanismus prädestiniert das erfindungsgemäße Tilirosid bzw. die erfindungsgemäße Zusammensetzung für einen prophylaktischen Einsatz, da bereits das Eindringen des Influenzavirus in die Wirtszelle ggf. verhindert wird.

Nach einer alternativen Ausgestaltung dieses Erfindungsgegenstandes werden das Tilirosid und/oder die Zusammensetzung ca. 48 Stunden nach der Exposition an dem Influenzavirus verabreicht.

Bei der Influenzavirusgrippe treten erst ca. 48h nach der Infektion erste Krankheitssymptome auf. Nach den Erkenntnissen der Erfinder ist eine Wirksamkeit auch noch ca. zwei Tage nach der Infektion zu erwarten und zwar genau gegen die Viren, die zu diesem Zeitpunkt Zellen des Körpers infizieren wollen. Die erfindungsgemäße Ausgestaltung macht sich diese Erkenntnis vorteilhaft zu Nutze.

Weiterhin offenbart wird ein Verfahren zur Herstellung einer pharmazeutischen Zusammensetzung zur Prophylaxe und/oder Behandlung von Infektionen durch Influenzaviren, das folgende Schritte ausweist:
1. Bereitstellung von Tilirosid [Kaempferol-3-(6"-trans-p-coumaroyl)glucosid] und/oder ein Derivat hiervon, und
2. Formulierung von Tilirosid und/oder dem Derivat hiervon in einen pharmazeutisch akzeptablen Träger,
wobei das Tilirosid und/oder Derivat hiervon das erfindungsgemäße Tilirosid und/oder Derivat hiervon ist. Dieses Verfahren ist nicht Gegenstand der Erfindung.

Die Erfindung wird nun anhand von Ausführungsbeispielen näher erläutert, aus denen sich weitere Merkmale, Vorteile und Eigenschaften der Erfindung ergeben. Die Ausführungsbeispiele sind rein illustrativ und schränken die Reichweite der Erfindung nicht ein.

Dabei wird Bezug genommen auf die beiliegenden Figuren, in denen Folgendes dargestellt ist:
- Fig. 1: zeigt das Ausmaß der antiviralen Wirkung verschiedener getesteter Substanzen gegen Influenzavirus A/Regensburg/D6/2009/H1N1; Verbindung 22 ist ein Ausführungsbeispiel gemäß der vorliegenden Erfindung, während ale übrigen getesteten Verbindungen Referenzbeispiele sind.
- Fig. 2: zeigt das Ergebnis zur Bestimmung der Konzentration von Tilirosid, die für 50% der A549-, MDCKII- und Vero-Zellen toxisch ist (TC₅₀);
- Fig. 3: zeigt das Ergebnis zur Bestimmung der effektiven Konzentration von Tilirosid, bei der 50% der Influenzaviren der Stämme A/Regensburg/D6/2009/H1N1, A/Victoria/3/75/H3N3, A/Mississippi/3/2001/H1N1, und A/Mississippi/3/2001/Variante H1N1 gehemmt werden (EC₅₀);
- Fig. 4: zeigt das Ergebnis aus den Untersuchungen zum Wirkmechanismus von Tilirosid gegen Influenzaviren (A/Regensburg/D6/2009/H1N1).

### Ausführungsbeispiele

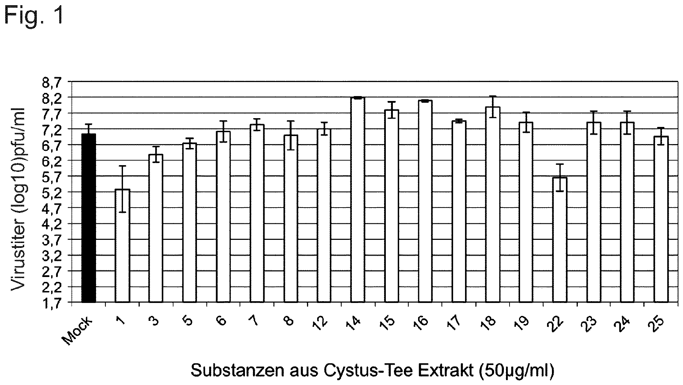

Folgende Substanzen wurden hinsichtlich ihrer antiviralen Wirkung untersucht: 1: Gallussäure; 3: Gallocatechin; 5: Gallocatechin-(4alpha-8)-catechin oder Catechin-(4alpha-8) gallocatechin; 6: Procyanidin B1 oder Procyanidin B3; 7: Procyanidin B1 oder Procyanidin B3; 8: Catechin; 12: Myricetin-3-*O*-galactosin; 14: Myricetin-*O*-xylosid; 15: Rutin und Myricitrin; 16: Quercetin-3-*O*-galactosid; 17: Quercetin-3-*O*-glucosid; 18: Quercetin-*O-*xylosid; 19: Quercitrin; 22: Tilirosid (trans-Isomer); 23: Tilirosid (Isomer); 24: Naringenin-7-methylether; 25: Apigenin-7-methy/lether. Das Ergebnis ist in der Fig. 1 dargestellt.

Dabei ist Verbindung 22 das erfindungsgemäße Tilirosid, während alle übrigen Verbindungen Referenzbeispiele sind.

Es zeigt sich, dass Tilirosid (Nr. 22) die höchste antivirale Aktivität aufweist und die Viruslast um mehr als 90% reduziert. Weiterhin konnte eine antivirale Aktivität bei der Gallussäure nachgewiesen werden.

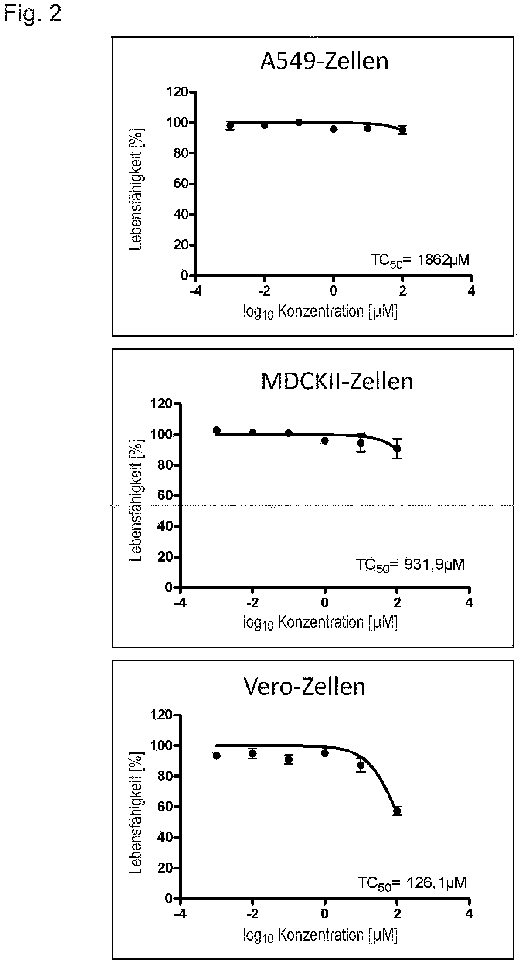

In einem nächsten Schritt wurde die Konzentration von Tilirosid bestimmt, die für 50% der A549-, MDCKII- und Vero-Zellen toxisch ist (TC₅₀). Das Ergebnis ist in der Fig. 2 gezeigt.

In A549-Zellen ergab sich ein TC₅₀-Wert von 1.862 µM. In MDCKII-Zellen ergab sich ein TC₅₀-Wert von 931,9 µM. In Vero-Zellen ergab sich ein TC₅₀-Wert von 126,1 µM. Aus diesen Ergebnissen lässt sich schließen, dass Tilirosid erst in relativ hohen Konzentrationen toxisch für die jeweilige Kulturzelle ist.

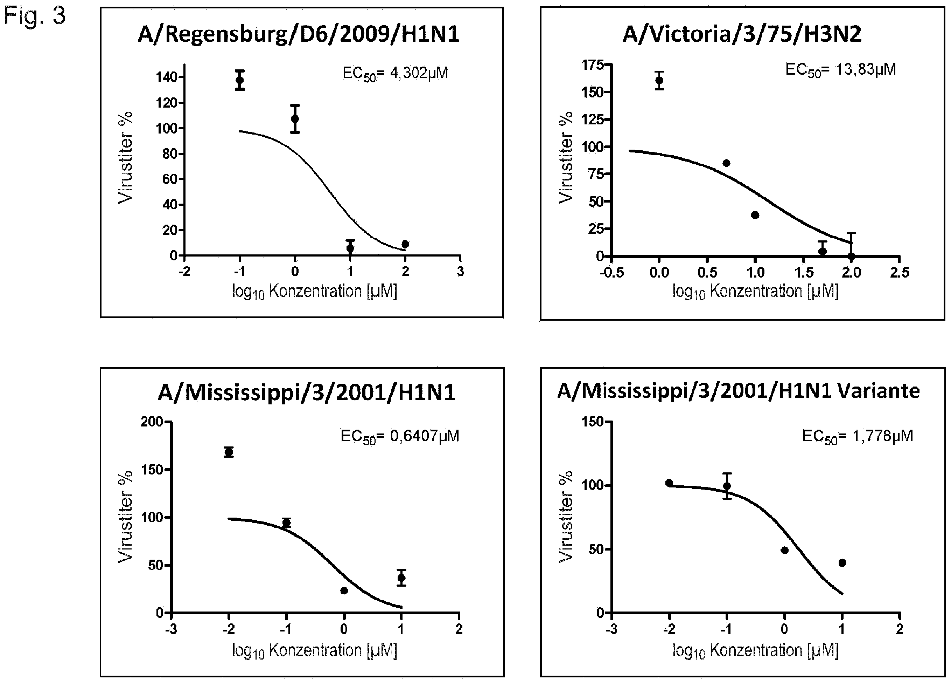

In einem weiteren Experiment wurde anhand von verschiedenen Influenza-Virusstämmen die Menge von Tilirosid bestimmt, die 50% inhibitorische Wirkung zeigt. Das Ergebnis ist in der Fig. 3 gezeigt.

Die Ergebnisse zeigen, dass Tilirosid gegen verschiedene Influenza-Virusstämme antiviral wirkt, auch gegen die Mississippi-Variante, die resistent gegen die antivirale Wirkung von Oseltamivir (Tamiflu) ist.

In weiteren Untersuchungen konnte gezeigt werden, dass Tilirosid nicht gegen Hantaviren wirkt (Daten nicht gezeigt). Dies belegt die spezifische Wirkung des Tilirosids.

Aus den TC₅₀/EC₅₀-Werten (siehe Fig. 2) lässt sich der Selektivitätsindex (S.I.) bestimmen. Je höher der Wert ist, desto potenter ist die antivirale Wirkung. Der S.I. für Tilirosid in diesen Experimenten beträgt gegen A/Regensburg/D6/2009/H1N1 = 433; A/Victoria/3/75/H3N2 = 134; A/Mississippi/3/2001/H1N1 = 2.906;
A/Mississippi/3/2001/Variante, H1N1 = 1.047. Dies zeigt, dass die antivirale Wirkung von Tilirosid sehr selektiv ist. Die Werte zeigen die Ratio an, wieviel mal Tilirosid überdosiert werden kann, bevor es per se für die jeweils verwendete Zelle toxisch wirkt.

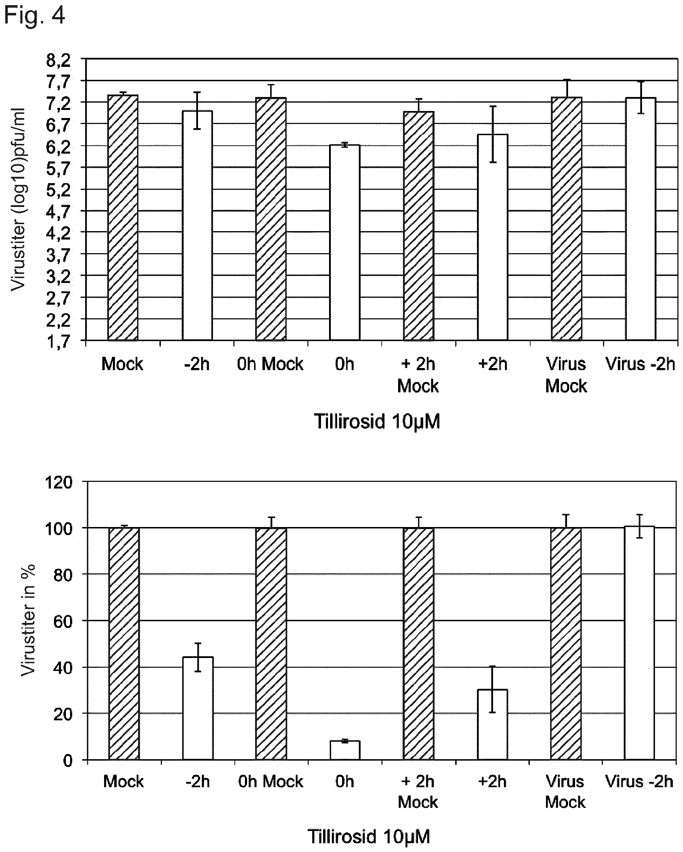

In einem weiteren Experiment sollte der Wirkmechanismus von Tilirosid aufgeklärt werden. Hierzu wurden Influenza-Viren vom Stamm A/Regensburg/D6/2009/H1N1 eingesetzt. Das Ergebnis ist in der Fig. 4 dargestellt.

Die Untersuchungen zeigen, dass Tilirosid die beste antivirale Wirkung aufweist, wenn die Behandlung zeitgleich mit der Infektion erfolgt (größer 90%). Beginnt die Behandlung zwei Stunden vor der Infektion, ist eine leichte Reduktion der Viruslast (kleiner 60%) zu beobachten. Eine Reduktion der Viruslast von ca. 70% ist zu beobachten, wenn Tilirosid zwei Stunden nach der Infektion zu den Zellen gegeben wird. Behandlungen, die vier Stunden nach Infektion begannen, zeigten keinen antiviralen Effekt (Daten nicht gezeigt).

Aus diesen Untersuchungen lässt sich schließen, dass Tilirosid einen Einfluss auf die Anheftung (Attachment) der Viren an die Wirtszelle oder auf die Internalisierung des Virus hat. Untersuchungen mit Hilfe der Western-Blot-Analyse bestätigen diese Hypothese. Deutlich wird aus den durchgeführten Experimenten, dass Tilirosid nicht "virostatisch" wirkt, da eine Vorinkubation der Viren mit Tilirosid zu keiner Reduktion der Viruslast führte.

### Fazit

Die Erfinder stellen erstmals eine Substanz bereit, die den Eintritt bzw. die Freisetzung der Influenzaviren in die Wirtszellen verhindert. Die Substanz ist hochselektiv und wirkt auch gegen solche Stämme, bei denen Oseltamivir aus dem Stand der Technik wirkungslos ist. Die von den Erfindern bereitgestellte Substanz stellt damit einen äußerst potenten Wirkstoff dar, der sich zur Prophylaxe und/oder Behandlung einer Infektion durch Influenzaviren besonders gut eignet.

## Patentansprüche

1. Tilirosid [Kaempferol-3-(6"-trans-p-coumaroyl)glucosid] zur Verwendung in der Prophylaxe und/oder Behandlung von Infektionen durch Influenzaviren.

2. Zusammensetzung zur Verwendung in der Prophylaxe und/oder Behandlung von Infektionen durch Influenzaviren, die Tilirosid [Kaempferol-3-(6"-trans-p-coumaroyl)glucosid] nach einem der Ansprüche 1 aufweist.

3. Zusammensetzung zur Verwendung nach Anspruch 2, **dadurch gekennzeichnet, dass** diese eine pharmazeutische Zusammensetzung ist, die zusätzlich eine pharmazeutische akzeptable Formulierung aufweist.

4. Zusammensetzung zur Verwendung nach Anspruch 2 oder 3, **dadurch gekennzeichnet, dass** diese in flüssiger, fester oder halb-fester Form vorliegt.

5. Zusammensetzung zur Verwendung nach einem der vorherigen Ansprüche, **dadurch gekennzeichnet, dass** diese zur oralen, topischen, rektalen oder vaginalen Verabreichung ausgebildet ist.

6. Zusammensetzung zur Verwendung nach einem der vorherigen Ansprüche, **dadurch gekennzeichnet, dass** diese in Form einer Tablette, Kapsel, eines Aerosols, Mundsprays, einer Mundspülung, Lösung, eines Pulvers, Granulats, Gels, einer Emulsion, Salbe, Paste, Tinktur, Creme ausgestaltet ist.

7. Tilirosid zur Verwendung nach Anspruch 1 und/oder der Zusammensetzung zur Verwendung nach einem der Ansprüche 2 bis 6 , **dadurch gekennzeichnet, dass** das Tilirosid und/oder die Zusammensetzung wie folgt verabreicht werden:
(i) unmittelbar vor der Exposition an dem Influenzavirus, und/oder
(ii) zeitgleich mit der der Exposition an dem Influenzavirus, und/oder
(iii) unmittelbar nach der Exposition an dem Influenzavirus.

8. Tilirosid zur Verwendug nach Anspruch 1 und/oder der Zusammensetzung zur Verwendung nach einem der Ansprüche 2 bis 6, **dadurch gekennzeichnet, dass** das Tilirosid und/oder die Zusammensetzung ca. 48 Stunden nach der Exposition an dem Influenzavirus verabreicht werden.

## Claims

1. Tiliroside [Kaempferol-3-(6"-trans-p-coumaroyl)glucoside] for use in prophylaxis and/or treatment of infections by influenza viruses.

2. The composition for use in prophylaxis and/or treatment of infections by influenza viruses, that exhibits Tiliroside [Kaempferol-3-(6"-trans-p-coumaroyl)glucoside] according to claim 1.

3. The composition for use according to claim 2, wherein this composition is a pharmaceutical composition which additionally exhibits a pharmaceutically acceptable formulation.

4. The composition for use according to claim 2 or 3, wherein the composition is present in liquid, solid or semi-solid Form.

5. The composition for use according to one of the preceding claims, wherein the composition is prepared for oral, topical, rectal, or vaginal administration.

6. The composition for use according to one of the preceding claims, wherein the composition is prepared in form of a tablet, capsule, aerosol, mouth rinse, solution, powder, granulate, gel, emulsion, ointment, paste, tincture, creme.

7. Tiliroside for use according to claim 1 and/or the composition for use according to claim 2 to 6, wherein the Tiliroside or the composition is administrated as follows:
(i) immediately before exposure with the influenza virus, and/or
(ii) at the same time as the exposure with the influenza virus, and/or
(iii) immediately after exposure with the influenza virus.

8. Tiliroside for use according to claim 1 and/or the composition for use according to claim 2 to 6, wherein the Tiliroside and/or the composition are administrated approx. 48 hours after exposure with the influenza virus.

## Revendications

1. Tiliroside [kaempférol-3-(6"-trans-p-coumaroyl)glucoside], destiné à être utilisé pour la prophylaxie et/ou le traitement d'infections par des virus d'Influenza.

2. Composition destinée à être utilisée pour la prophylaxie et/ou le traitement d'infections par des virus d'Influenza contenant du tiliroside [kaempférol-3-(6"-trans-p-coumaroyl)glucoside] selon l'une quelconque des revendications 1.

3. Composition destinée à être utilisée selon la revendication 2, **caractérisé en ce que** celle-ci est une composition pharmaceutique comportant en outre une formulation pharmaceutiquement acceptable.

4. Composition destinée à être utilisée selon la revendication 2 ou 3, **caractérisé en ce qu'**elle existe sous forme liquide, solide ou semisolide.

5. Composition destinée à être utilisée selon l'une quelconque des revendications précédentes, **caractérisé en ce qu'**elle est conçue pour l'administration par voie orale, topique, rectale ou vaginale.

6. Composition destinée à être utilisée selon l'une quelconque des revendications précédentes, **caractérisé en ce qu'**elle est conçue sous forme d'un comprimé, d'une capsule, d'un aérosol, d'une pulvérisation buccale, d'un bain de bouche, d'une solution, d'une poudre, de granulés, d'un gel, d'une émulsion, d'une pommade, d'une pâte, d'une teinture, d'une crème.

7. Tiliroside destiné à être utilisé selon la revendication 1 et/ou la composition destinée à être utilisée selon l'une quelconque des revendications 2 à 6, **caractérisé en ce que** le tiliroside et/ou la composition sont administrés comme suit:
(i) immédiatement avant l'exposition au virus d'Influenza, et/ou
(ii) simultanément avec l'exposition au virus d'Influenza, et/ou
(iii) immédiatement après l'exposition au virus d'Influenza.

8. Tiliroside destiné à être utilisé selon la revendication 1 et/ou la composition destinée à être utilisée selon l'une quelconque des revendications 2 à 6, **caractérisé en ce que** le tiliroside et/ou la composition sont administrés environ 48 heures après l'exposition au virus d'Influenza.
